(19) Europäisches Patentamt
European Patent Office
Office européen des brevets

(11) Veröffentlichungsnummer : **0 018 576**
**B1**

(12) # EUROPÄISCHE PATENTSCHRIFT

(45) Veröffentlichungstag der Patentschrift : **28.04.82**

(51) Int. Cl.³ : **C 07 C 85/06**, C 07 C 87/62

(21) Anmeldenummer : **80102153.6**

(22) Anmeldetag : **22.04.80**

(54) **Verfahren zur Herstellung von N-alkylierten aromatischen Aminen.**

(30) Priorität : **04.05.79 DE 2918023**

(43) Veröffentlichungstag der Anmeldung : **12.11.80 (Patentblatt 80/23)**

(45) Bekanntmachung des Hinweises auf die Patenterteilung : **28.04.82 Patentblatt 82/17**

(84) Benannte Vertragsstaaten : **BE CH DE FR GB IT LI NL**

(56) Entgegenhaltungen :
**DE - B2 - 2 348 738**
**DE - C - 862 156**
**DE - C - 1 003 220**
**GB - A - 1 358 816**
**HOUBEN-WEYL « Methoden der organischen Chemie » Band XI/1 1962, GEORG THIEME VERLAG, Stuttgart, Seiten 134 bis 143**

(73) Patentinhaber : **BAYER AG**
**Zentralbereich Patente, Marken und Lizenzen**
**D-5090 Leverkusen 1, Bayerwerk (DE)**

(72) Erfinder : **Schulte-Huermann, Werner, Dr.**
**Helmut-Macke-Strasse 10**
**D-4150 Krefeld-Bockum (DE)**
Erfinder : **Hemmerich, Heinz Peter, Dr.**
**Bethelstrasse 14**
**D-4150 Krefeld-Bockum (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 018 576 B1**

Imprimerie Jouve, 18, rue St-Denis, 75001 Paris, France

# Verfahren zur Herstellung von N-alkylierten aromatischen Aminen

Die Erfindung betrifft ein Verfahren zur Herstellung von N-alkylierten aromatischen Aminen durch Umsetzung von aromatischen Aminen mit aliphatischen oder cycloaliphatischen Alkoholen in Gegenwart von Phosphoroxihalogeniden.

Aus Houben-Weyl, Methoden der organischen Chemie, Band XI/1, Seiten 134 bis 143, Thieme, Stuttgart 1962 ist die N-Alkylierung aromatischer Amine mit Alkoholen in Gegenwart von Reaktionsbeschleunigern bekannt. Als Reaktionsbeschleuniger werden anorganische Säuren wie Halogenwasserstoffsäuren, Schwefelsäure, Phosphorsäure sowie Metallsalze wie Zinkchlorid, Eisen-(III)-chlorid oder Kupfer-(II)-chlorid genannt. Daneben können auch Jod, Bortrifluorid und Phosphortrichlorid verwendet werden.

Nachteilig ist bei Einsatz der besonders aktiven Katalysatoren wie der Halogenwasserstoffsäuren, des Phosphortrichlorids oder des elementaren Jods deren starke korrosive Wirkung auf die aus Eisen oder Eisenlegierungen bestehenden Apparate und Rohrleitungen.

Es wurde ein Verfahren zur Herstellung von N-alkylierten aromatischen Aminen durch Umsetzung von aromatischen Aminen mit aliphatischen oder cycloaliphatischen Alkoholen bei erhöhter Temperatur und Druck in Gegenwart von Katalysatoren gefunden, das dadurch gekennzeichnet ist, daß man die Umsetzung in Gegenwart von Phosphoroxihalogeniden durchführt.

Als Phosphoroxihalogenide, die in das erfindungsgemäße Verfahren eingesetzt werden können, seien z.B. genannt : Phosphoroxichlorid und Phosphoroxibromid. Bevorzugt wird Phosphoroxichlorid eingesetzt.

Die Phosphoroxihalogenide können allein oder im Gemisch untereinander in das erfindungsgemäße Verfahren eingesetzt werden.

Im allgemeinen werden die Phosphoroxihalogenide in einer Menge von etwa 0,05 bis etwa 2,0 Gew.-%, vorzugsweise in einer Menge von etwa 0,1 bis etwa 1,0 Gew.-%, bezogen auf eingesetztes aromatisches Amin und eingesetzten aliphatischen oder cycloaliphatischen Alkohol in das erfindungsgemäße Verfahren eingesetzt.

Als aromatische Amine können solche der allgemeinen Formel I

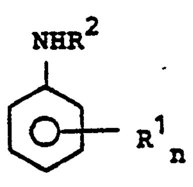

(I)

in der

$R^1$ für Halogen, einen niederen Alkyl-, Alkoxy- oder Alylmercaptorest,

$R^2$ für Wasserstoff oder einen niederen Alkylrest steht und

n eine der Zahlen 0, 1, 2 oder 3 bedeutet, wobei im Falle, daß n für 2 oder 3 steht, die Reste $R^1$ auch verschieden sein können,

in das erfindungsgemäße Verfahren eingesetzt werden.

Weiterhin können als aromatische Amine solche der allgemeinen Formel II

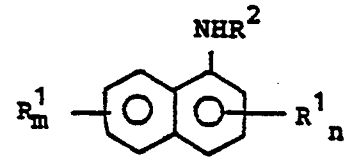

(II)

in der

$R^1$ und $R^2$ die in Formel (I), sowie n und m unabhängig voneinander die in Formel (I) für n angegebene Bedeutung haben,

in das erfindungsgemäße Verfahren eingesetzt werden.

Als Halogene seien Fluor, Chlor, Brom und Jod, bevorzugt Chlor und Brom, genannt.

Als niedere Alkylreste seien solche mit bis zu 4 C-Atomen, bevorzugt bis zu 2 C-Atomen, genannt ; beispielsweise der Methyl-, Ethyl-, Propyl-, Butyl-Rest, bevorzugt der Methyl-, Ethyl-Rest.

Als Alkoxyreste kommen solche mit bis zu 4 C-Atomen, bevorzugt mit bis zu 2 C-Atomen, in Frage ; z.B. seien genannt der Methoxy-, Ethoxy-, Propoxy-, Butoxy-Rest, bevorzugt der Methoxy-, Ethoxy-Rest.

Als Alkylmercaptoreste seien solche mit bis zu 4 C-Atomen, bevorzugt mit bis zu 2 C-Atomen, genannt ; beispielsweise der Methylmercapto-, Ethylmercapto-, Propylmercapto-, Butylmercapto-Rest, bevorzugt der Methylmercapto-, Ethylmercapto-Rest.

Besonders bevorzugt werden solche aromatische Amine der Formel (I) und (II) in das erfindungsgemäße Verfahren als Ausgangsmaterial eingesetzt, in denen $R^1$ für Chlor, Methyl oder Ethyl und $R^2$ für Wasserstoff, Methyl oder Ethyl stehen und n, m=0, 1 oder 2, insbesondere 0 oder 1, sind.

Als beispielhafte Vertreter für die aromatischen Amine der Formel (I) und (II), die nach dem erfindungsgemäßen Verfahren alkyliert werden können, seien genannt :

Anilin, 1-Naphthylamin, o-, m-, p-Toluidin, o-, m-, p-Ethylanilin, die Xylidine, wie 2,3-, 2,4-, 2,5-, 2,6-, 3,4-, 3,5-Dimethylanilin, 4-Methyl-naphthylamin-1, die Chlor-substituierten Aniline wie 2-Chlor-, 3-Chlor- und 4-Chlor-anilin, 3-Chlor-o-, 4-Chlor-o-, 5-Chlor-o-, 4-Chlor-m, 6-Chlor-m-, 2-Chlor-p- und 3-Chlor-p-toluidin, 4-Chlor- und 5-Chlor-naphthylamin-1.

In das erfindungsgemäße Verfahren können grundsätzlich alle aliphatischen oder cycloaliphatischen Alkohole eingesetzt werden, sofern sie die Bedingung hinreichender Stabilität unter den Reaktionsbedingungen erfüllen.

Bevorzugt sind aliphatische Alkohole mit 1 bis 10 Kohlenstoffatomen und cycloaliphatische Alkohole mit 4 bis 10 Kohlenstoffatomen, besonders bevorzugt solche aliphatische Alkohole

mit 1 bis 6 Kohlenstoffatomen und cycloaliphatische Alkhole mit 5 bis 8 Kohlenstoffatomen.

Es seien z.B. genannt :

Methanol, Ethanol, n-Propanol, i-Propanol, n-Butanol, i-Butanol, tert.-Butanol, n-Pentanol, i-Pentanol, Hexanol, Heptanol, Octanol, Cyclobutanol, -pentanol, -hexanol, -heptanol, -octanol, 2-Methyl-, 3-Methyl-, 4-Methyl-cyclohexanol, 2-Ethyl-cyclohexanol, 3.3.5-Trimethylcyclohexanol, 4-tert.-Butylcyclohexanol, Menthol.

Besonders bevorzugt seien genannt :

Methanol, Ethanol, n-Propanol, i-Propanol, n-Butanol, Cyclohexanol.

Das Molverhältnis von aromatischem Amin zu aliphatischen oder cycloaliphatischen Alkoholen kann in weiten Grenzen schwanken. Im allgemeinen liegt das Molverhältnis von aromatischem Amin zu aliphatischem oder cycloaliphatischem Alkohol im Bereich von etwa 1 : 0,5 bis etwa 1 : 20, bevorzugt bei 1 : 0,7 bis 1 : 5.

Das erfindungsgemäße Verfahren kann bei Temperaturen im Bereich von etwa 200 bis 400 °C, vorzugsweise bei 230 bis 320 °C, durchgeführt werden.

Die Drücke betragen im allgemeinen etwa 30 bis etwa 200 bar, bevorzugt 80 bis 150 bar.

Die Reaktionszeit ist abhänig von den Einsatzkomponenten, der Katalysatorkonzentration, den Reaktionsbedingungen wie Druck und Temperatur und dem gewünschten Umsetzungsgrad.

Im allgemeinen liegen die Reaktionszeiten im Bereich von etwa 1 bis etwa 10 Stunden.

Die erfindungsgemäß eingesetzten Phosphoroxihalogenide besitzen eine hohe Katalysatoraktivität bei der N-Alkylierung von aromatischen Aminen mit aliphatischen oder cycloaliphatischen Alkoholen, wobei es besonders überraschend ist, daß die erfindungsgemäß eingesetzten Phosphoroxihalogenide im Gegensatz zu den bekannten Katalysatoren kaum korrosiv unter den Reaktionsbedingungen genüber den üblicherweise aus Eisen oder Eisenlegierungen bestehenden Apparaturen und Rohrleitungen wirken.

Darüber hinaus verfügen die Phosphoroxihalogenide über die Eigenschaft, weder im Gemisch der Ausgangskomponenten noch in der ausreagierten Mischung feste Bestandteile zu bilden, was für die kontinuierliche Durchführung der N-Alkylierung von aromatischen Aminen außerordentlich wichtig ist.

Schließlich hat Phosphoroxichlorid im Gegensatz zu Phosphortrichlorid einen bedeutenden Vorteil für die Arbeitssicherheit, da das Phosphoroxichlorid im Alkylierungsgemisch (aromatisches Amin plus Alkohol) bei Kontakt mit Wasser nicht selbstentzündlich ist.

Die nachstehend aufgeführten Beispiele dienen zur Erläuterung des erfindungsgemäßen Verfahrens ohne es jedoch auf die Beispiele einzuschränken.

Beispiel 1

In einem 3 l Autoklaven mit Hubrüher wird ein Gemisch aus 1 860 g Anilin, 512 g Methanol und 3,1 g $POCl_3$ vorgelegt. Nach dem Verschließen wird der Autoklav auf 280 °C hochgeheizt und ca. 3 Stunden bei dieser Temperatur gehalten. Dabei stellt sich ein Druck von etwa 80 bar ein.

Man erhält ein Rohöl, das nach dem Gaschromatogramm folgende Zusammensetzung (Methanol bleibt unberücksichtigt) hat : 39 % Anilin, 43 % N-Methylanilin und 17 % N.N-Dimethylanilin.

Nach Neutralisation mit verdünnter Natronlauge, Abdestillieren des nicht umgesetzten Methanols und Abtrennen der wäßrigen Schicht wurde das Rohprodukt über eine 150 cm. — Füllkörperkolonne mit verspiegeltem Vakuummantel bei einem Rücklaufverhältnis von etwa 20 fraktionierend destilliert. Es wurden 850 g eines Hauptlaufes abgenommen, der laut gaschromatographischer Analyse aus 98.8 % N-Methylanilin, 1.1 % N.N-Dimethylanilin und Spuren Anilin bestand.

Beispiel 2

Der Ansatz aus Beispiel 1 wird mit 6 g $POBr_3$ wiederholt. Die Rohölzusammensetzung beträgt : 35 % Anilin, 46 % N-Methylanilin, 18 % N.N-Dimethylanilin.

Beispiel 3

In einem 3 l Autoklaven mit Hubrührer wird ein Gemisch aus 749 g p-Toluidin, 1 008 g Methanol und 7,9 g $POCl_3$ vorgelegt. Nach dem Verschließen wird der Autoklav auf 280 °C hochgeheizt und ca. 3 Stunden bei dieser Temperatur gehalten. Dabei stellt sich ein Druck von etwa 60 bar ein. Man erhält ein Rohöl der Zusammensetzung < 1 % p-Toluidin, 1 % N-Methyl-p-toluidin und 94 % N.N-Dimethyl-p-toluidin.

Beispiel 4

Bei gleicher Verfahrensweise wie im Beispiel 3 wird ein Gemisch aus 1 116 g Anilin, 607 g Ethanol und 4,2 g $POCl_3$ eingesetzt. Die Rohölzusammensetzung beträgt : 31 % Anilin, 61 % N-Ethylanilin, 8 % N.N-Diethylanilin.

Beispiel 5

Bei gleicher Verfahrensweise wie im Beispiel 3 wird ein Gemisch aus 1 284 g o-Toluidin, 607 g Ethanol und 6,3 g $POCl_3$ eingesetzt. Die Rohölzusammensetzung beträgt : 33 % o-Toluidin, 63 % N-Ethyl-o-toluidin, 3 % N.N-Diethyl-o-toluidin.

Beispiel 6

Bei gleicher Verfahrensweise wie in Beispiel 3 wird ein Gemisch aus 1 285 g m-Toluidin, 720 g i-Propanol und 6,7 g $POCl_3$ eingesetzt. Die Rohölzusammensetzung beträgt : 53 % m-Toluidin, 45 % N-Isopropyl-m-toluidin, 1 bis 2 % N.N-Diisopropyl-m-toluidin.

Beispiel 7

Bei gleicher Verfahrensweise wie in Beispiel 3 wird ein Gemisch aus 1 023 g Anilin, 814 g n-Butanol und 3,7 g $POCl_3$ eingesetzt. Die Rohölzusammensetzung beträgt : 43 % Anilin, 53 % N-n-Butylanilin, 3 % N.N-Di-n-butylanilin.

Beispiel 8

Bei gleicher Verfahrensweise wie in Beispiel 3 wird ein Gemisch aus 931 g Anilin, 1 002 g Cyclohexanol und 3,8 g $POCl_3$ eingesetzt. Rohölzusammensetzung : 73 % Anilin, 22 % N-Cyclohexylanilin, 1-2 % N.N-Dicyclohexylanilin. Es treten nennenswerte Cyclohexanolverluste durch Cyclohexenbildung auf.

Beispiel 9

Bei gleicher Verfahrensweise wie in Beispiel 3 wird ein Gemisch aus 931 g 1-Naphthylamin, 897 g Ethanol und 8,2 g $POCl_3$ eingesetzt. Rohölzusammensetzung : 30 % 1-Naphthylamin, 52 % N-Ethyl-1-naphthylamin, 6 % N.N-Diethyl-1-naphthylamin.

Beispiel 10

In einer Anlage zur kontinuierlichen Hochdruckalkylierung von Anilin mit Methanol zu N-Methyl- und N.N-Dimethylanilin wurden Korrosionsproben aus den Edelstählen Werkstoff 1.4571 und 1.4439 eingebracht. Die Reaktionsbedingungen wurden konstant gehalten und waren im einzelnen wie folgt : Molverhältnis Anilin : Methanol = 1 : 1 und 0,117 % $POCl_3$, bezogen auf Alkylierungsgemisch. Reaktionstemperatur 270 °C, Druck 100 bar.

Nach einer Fahrzeit von 36 Tagen wurde der Korrosionsabtrag bestimmt :

| | |
|---|---|
| Werkstoff 1.4571 | 0,235 mm/Jahr, |
| Werkstoff 1.4439 | 0,307 mm/Jahr. |

Vergleichsbeispiel

Der Korrosionstest nach Beispiel 10 wurde unter gleichen Bedingungen wiederholt, jedoch mit Einsatz von 0,1 % $PCl_3$, bezogen auf Alkylierungsgemisch.

Nach einer Fahrzeit von 35 Tagen wurde erneut der Korrosionsabtrag bestimmt :

| | |
|---|---|
| Werkstoff 1.4571 | 1,144 mm/Jahr, |
| Werkstoff 1.4439 | 2,096 mm/Jahr. |

**Ansprüche**

1. Verfahren zur Herstellung von N-alkylierten aromatischen Aminen durch Umsetzung von aromatischen Aminen mit aliphatischen oder cycloaliphatischen Alkoholen bei erhöhter Temperatur und Druck in Gegenwart von Katalysatoren, dadurch gekennzeichnet, daß man die Umsetzung in Gegenwart von Phosphoroxihalogeniden durchführt.

2. Verfahren nach Anspruch 1, dadurch gekennzeichnet, daß man als Phosphoroxihalogenide Phosphoroxichlorid und/oder Phosphoroxibromid einsetzt.

3. Verfahren nach Ansprüchen 1 und 2, dadurch gekennzeichnet, daß man als Phosphoroxihalogenid Phosphoroxichlorid einsetzt.

4. Verfahren nach Ansprüchen 1 bis 3, dadurch gekennzeichnet, daß man die Phosphoroxihalogenide in Mengen von 0,05 bis 2,0 Gew.-%, bezogen auf eingesetztes aromatisches Amin und eingesetzten aliphatischen oder cycloaliphatischen Alkohol, einsetzt.

**Claims**

1. Process for the preparation of N-alkylated aromatic amines by reacting aromatic amines with aliphatic or cycloaliphatic alcohols at elevated temperature and under increased pressure, in the presence of catalysts, characterised in that the reaction is carried out in the presence of phosphorus oxyhalides.

2. Process according to Claim 1, characterised in that phosphorus oxychloride and/or phosphorus oxybromide are employed as the phosphorus oxyhalides.

3. Process according to Claims 1 and 2, characterised in that phosphorus oxychloride is employed as the phosphorus oxyhalide.

4. Process according to Claims 1 to 3, characterised in that the phosphorus oxyhalides are employed in amounts of 0,05 to 2.0 % by weight, relative to aromatic amine employed and to aliphatic or cycloaliphatic alcohol employed.

**Revendications**

1. Procédé pour la préparation d'amines aromatiques N-alkylées par réaction d'amines aromatiques avec des alcools aliphatiques ou cycloaliphatiques à température et sous pression élevées en présence de catalyseurs, caractérisé en ce que l'on effectue la réaction en présence d'oxyhalogénures de phosphore.

2. Procédé selon la revendication 1, caractérisé en ce que l'on utilise comme oxyhalogénures de phosphore l'oxychlorure de phosphore et/ou l'oxybromure de phosphore.

3. Procédé selon les revendications 1 et 2, caractérisé en ce que l'on utilise comme oxyhalogénure de phosphore l'oxychlorure de phosphore.

4. Procédé selon les revendications 1 à 3, caractérisé en ce que l'on utilise les oxyhalogénures de phosphore en quantités de 0,05 à 2,0 % en poids, par rapport à l'amine aromatique utilisée et à l'alcool aliphatique ou cycloaliphatique utilisé.